# EUROPEAN PATENT SPECIFICATION

(11) **EP 4 561 664 B1**
(45) Date of publication and mention of the grant of the patent: **08.04.2026**
(21) Application number: 23744242.1
(22) Date of filing: 05.07.2023
(51) Int. Cl.: A61B 5/00, A61B 5/01, A61B 5/021, A61B 5/024, A61B 5/145, A61B 5/1455, A61M 60/113, A61M 60/20, A61M 60/882, A61M 60/36, A61N 1/378, G16H 40/63, H01M 16/00, H01M 50/247, H01M 8/16

(54) **MEDICAL DEVICE AND MEDICAL SYSTEM USING SAID MEDICAL DEVICE**
MEDIZINISCHES GERÄT UND MEDIZINISCHES SYSTEM, DAS DIESES MEDIZINISCHE GERÄT VERWENDET
DISPOSITIF MÉDICAL ET SYSTÈME MÉDICAL UTILISANT CE DISPOSITIF MÉDICAL

(30) Priority: 26.07.2022 IT 202200015768
(43) Date of publication of application: 04.06.2025
(73) Proprietor: De Asmundis, Carlo, 1653 Dworp (BE); Talevi, Giacomo, 60015 Falconara Marittima (AN) (IT); Mali, Alvja, 60015 Falconara Marittima (AN) (IT)
(72) Inventor: De Asmundis, Carlo, 1653 Dworp (BE); Talevi, Giacomo, 60015 Falconara Marittima (AN) (IT); Mali, Alvja, 60015 Falconara Marittima (AN) (IT)
(74) Representative: Casalonga
(86) International application number: PCT/IB2023/056945
(87) International publication number: WO 2024/023610

(56) References cited:
- US-A1- 2012 283 968
- US-A1- 2018 233 761
- US-B1- 9 647 289

## Description

### TECHNICAL FIELD

The present invention relates to a medical device and a medical system using said medical device. A method for recharging said medical device and said medical system are further described. The medical device and medical system may be used in the field of medical instrumentation, such as for monitoring and caring of diabetic and cardiac patients.

### BACKGROUND ART

Medical devices for monitoring the state of health of a patient are known in the art; these devices may be either implantable or extracorporeal, namely they may be applied to the patient's skin.

For example, there are known implantable medical devices equipped with a glucose sensor and a wireless transmitter suitable for transmitting data collected by the sensor to an external mobile device, through which the patient himself or trained medical personnel monitor blood glucose levels to prevent hyperglycemic or hypoglycemic conditions. Implantable medical systems that use biofuel cells to generate electricity are also known; see, for example, patent applications No. US 2012/283968 A1 and No. US 2018/233761.

Although such devices are used today, for example, to monitor a patient, the Applicant noted that they require a highly invasive installation and maintenance procedure for the patient.

Extracorporeal medical devices for glucose monitoring, applied to a patient's skin, are also known; such devices have an external glucose sensor and a wireless transmitter suitable for transmitting data collected by the sensor to a mobile device. Unlike implantable devices, extracorporeal medical devices do not require invasive installation and maintenance procedures. The Applicant noted, however, that even such known medical devices require constant and careful maintenance by the patient; for this reason, known external medical devices are unreliable in that, proper functioning is entirely up to the maintenance performed by the patient himself. In fact, in the event that constant and careful maintenance is not performed by the patient, the medical device may not function properly, thus preventing the proper monitoring of the patient's health status.

An additional extracorporeal medical device is described in U.S. Patent No. US 9 647 289 B1. Such device includes a container that may be attached to a patient and configured to receive a flow of blood; in the container there is a solution that, when in contact with a patient's blood, allows to lower the patient's blood sugar level.

It is also known from U.S. patent application No. US 2019/260053 A1 a medical device comprising a biofuel cell using the electro-oxidation of blood glucose to produce electricity, which is used to power the medical device itself. In detail, the medical device includes a rechargeable battery that is electrically connected to the biofuel cell and configured to recharge from the energy produced by the biofuel cell. The medical device is also equipped with a pump used to deliver a dose of insulin into the patient upon the occurrence of a hyperglycemic condition or to deliver glucose upon the occurrence of a hypoglycemic condition. Although the solution described in the U.S. patent application reduces the need for battery replacement due to the presence of the biofuel battery, the Applicant noted that the device described in application No. US 2019/260053 A1 is not without drawbacks. In fact, such medical device requires the use of two different reservoirs, one containing insulin and one containing a glucose solution, giving the device a high structural complexity and a large footprint. Additionally, the Applicant noted that the presence of the reservoirs (insulin and glucose) requires more maintenance, a condition that makes the device unsafe.

### OBJECT OF THE INVENTION

The object of the present invention is to solve at least one of the drawbacks and/or limitations of the previous solutions. It is an object of the present invention to provide a non-invasive medical device that is easy to apply, at the same time capable of operating for long periods of time without the need for maintenance by a trained medical professional or by the patient himself. It is then object of the present invention to provide a medical device suitable for performing precise monitoring of parameters in the blood and ensuring optimal health conditions for the patient. It is also object of the present invention to provide a medical device having a simple and compact structure, which has low production costs; in particular, it is object of the present invention to provide a mobile extracorporeal device, easily applicable on a patient and thus easily transportable. It is also an object to provide a simple and compact medical system that minimizes the maintenance of the system itself.

These objects and others, which will appear from the following description, are substantially achieved by a medical device and a medical system according to one or more of the following claims and/or aspects.

### BRIEF DESCRIPTION OF THE DRAWINGS

Some embodiments and some aspects of the invention will be described below with reference to the accompanying drawings, provided for illustrative purposes only and therefore not limiting wherein:
- Figure 1 is a schematic view of a medical device applied on a patient's skin;
- Figure 2 is a schematic view of the medical device of Figure 1;
- Figure 3 is a schematic view of a medical system including a medical device according to the present invention;
- Figure 4 is a schematic view of a variant of a medical system including a medical device according to the present invention;

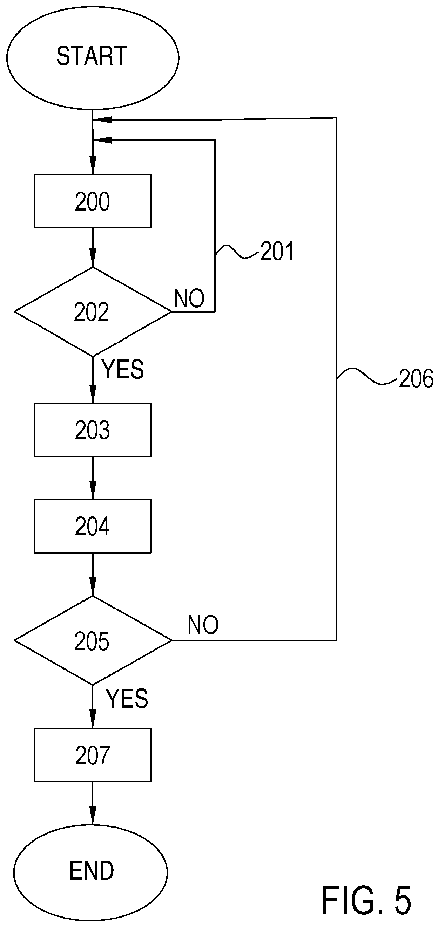

- Figure 5 is a flow chart showing the steps implementable by a medical device according to the present invention;
- Figure 6 is a further schematic view of a medical device according to the present invention;

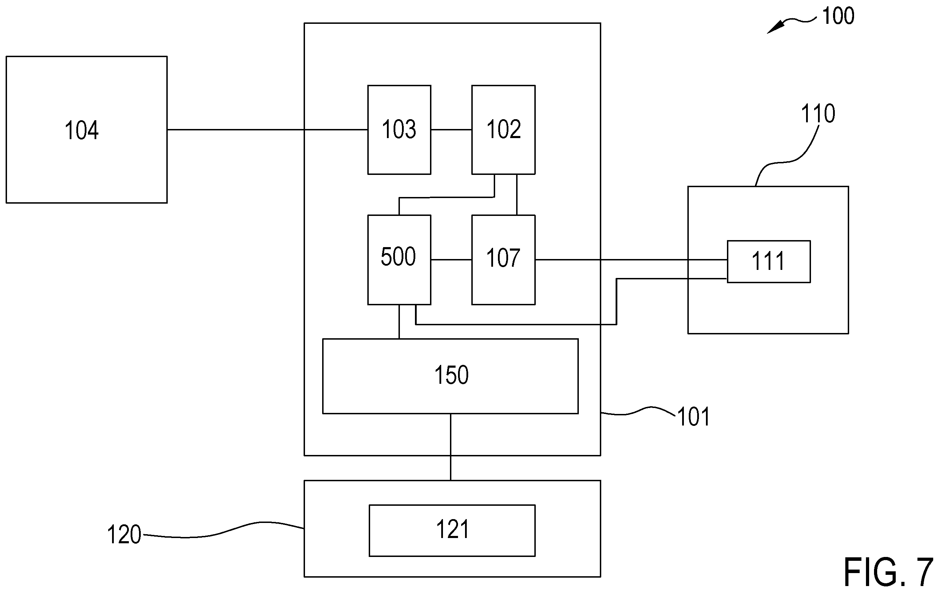

- Figures 7 and 8 are respective schematic views of variations of the medical system of Figure 4;
- Figure 9 is a flow chart showing the steps implementable by a medical system.

### DEFINITIONS AND CONVENTIONS

Note that in the present detailed description the parts illustrated in the various figures are shown with the same numerical references. The figures may illustrate the subject matter of the invention by means of representations that are not to scale; therefore, parts and components illustrated in the figures related to the subject matter of the invention may relate only to schematic representations.

At least one of the medical device, implantable device, and medical system described below may include/use at least one control unit responsible for controlling operating conditions performed by the same medical device, implantable device, or the medical system and/or for controlling method steps executable by them. The control unit may be a single unit or consist of a plurality of distinct control units depending on design choices and operational requirements.

By control unit is meant an electronic component which may include at least one of: a digital processor (CPU), an analog type circuit, or a combination of one or more digital processors with one or more analog type circuits. The control unit may be *"configured"* or *"programmed"* to perform certain steps: this may be accomplished in practice by any means that allows the control unit to be configured or programmed. For example, in the case of a control unit comprising one or more CPUs and one or more memories, one or more programs may be stored in appropriate memory banks attached to the CPU(s); the program(s) contain instructions that, when executed by the CPU(s), program or configure the control unit to perform the operations described in relation to the control unit. Alternatively, if the control unit is/includes analog type circuitry, then the circuitry of the control unit may be designed to include circuitry configured, in use, to process electrical signals such that to perform the steps related to the control unit. Portions of the process described herein may be accomplished by means of a data processing unit, or control unit, that is technically replaceable with one or more electronic processors designed to execute a portion of software program or firmware loaded into a memory medium. Such software program may be written in any programming language of known type. The electronic processors, if two or more in number, may be interconnected by means of a data connection such that their computational powers are shared; the same electronic processors may thus be installed in geographically different locations, realizing through the aforementioned data connection a distributed computing environment. The data processing unit, or control unit, may be a *general purpose* processor configured to perform one or more parts of the process identified in the present disclosure through the software program or firmware, or may be an ASIC or dedicated processor or FPGA, specifically programmed to perform at least part of the operations of the process described herein. The memory medium may be non-transitory and may be internal or external to the processor, or control unit, or data processing unit, and specifically may be a memory geographically located remote from the processor. The memory medium may also be physically divided into multiple portions, or in *cloud* form, and the software program or firmware may be stored on geographically divided portions of memory.

The biofuel cell is configured to produce electricity through the chemical transformation of a biological substrate. The biofuel cell used in the medical device 1 and/or medical system 100 may be of enzymatic type i.e., using enzymes as catalysts for allowing the occurrence of chemical reactions for producing electricity. The enzymatic biofuel cell may perform a chemical transformation to obtain electricity under physiological reaction conditions (in the range of 37°C and basically under pH-7 conditions). The enzymes usable in the biofuel cell are: glucose oxidase (GOx), glucose dehydrogenase (GDH), bilirubin oxidase (BOD), and laccase. The biofuel cell may take advantage of direct electron transfer (DET) through the use of nanostructures, in particular, carbon nanostructures (CNTs). The electrodes (anode and cathode) of the biofuel cell may be arranged in porous bodies, communicating with the blood through a selective membrane (e.g., a glucose selective membrane for the anode and an oxygen selective membrane for the cathode). The electrodes may also be separated from each other, on the inner side, that is, on the side where the electrodes face each other, by a selective membrane for ions (H⁺) that, by osmosis, may pass from the anode to the cathode for the formation of water.

The term *'implantable'* refers to a device and/or system that may be at least partially implanted under a patient's skin.

### DETAILED DESCRIPTION

### Medical device

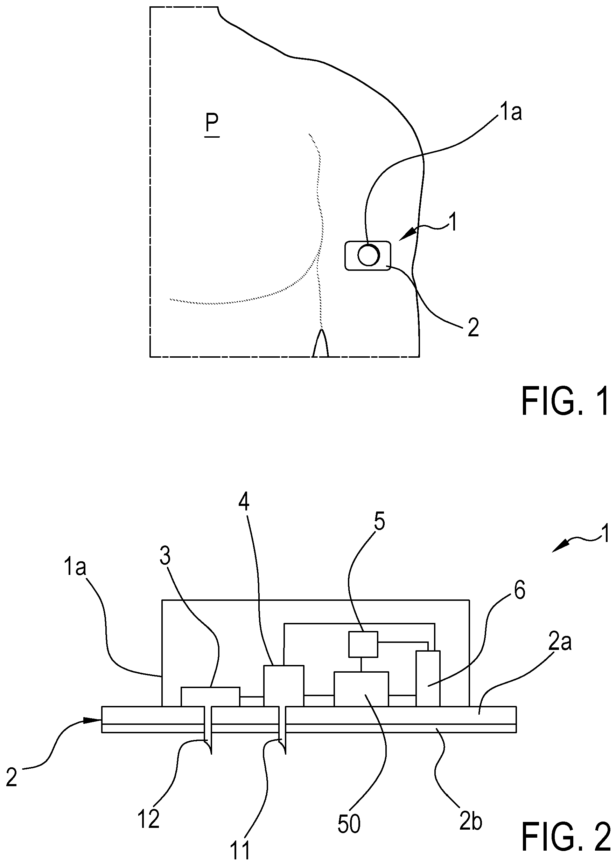

A medical device applicable to the skin of a patient P has been collectively referred to as 1. The device uses one or more substances in the fluid of the patient (optionally biological fluid) to produce electricity. The medical device 1 is designed for external application and, as shown in Figure 1, may include a holder 2, such as a plaster, applicable on the skin of patient P over a large, linear area, such as the torso, back, arms, and thighs. To produce electricity, the medical device 1 monitors and exploits one or more substances, such as glucose, through a noninvasive technique that allows data acquisition without the need to interact directly with the biological environment of the patient.

The holder 2 may have a multilayer structure, for example defined by at least one or more upper or rigid layers 2a, overlapped to a lower layer or adhesive layer 2b suitable for contacting the skin of the patient. The adhesive layer 2b may be made of elastic and flexible material to fit as closely as possible to the skin surface where it is applied to maximize adherence. Each rigid layer is carried by the adhesive layer on the side opposite the skin, for supporting one or more of the subsequently detailed electrical or electronic components of the medical device 1.

The holder 2 may have a rectangular conformation (Figure 1) of a suitable size for allowing its application on the arms; for example, the holder 2 may have a contact surface suitable for being placed in contact with the skin of a patient having a surface area comprised between 20 cm² and 150 cm². In fact, the contact surface is essentially defined by the adhesive layer 2b. However, it is not excluded use of a holder 2 having a different shape (e.g., circular or square) or different dimensions, varying according to the application points of the medical device 1 itself. From a material point of view, the holder 2 may be made of sterile, skin-friendly and hypoallergenic material, e.g., cotton (in the case of adhesive layer 2b) or rigid synthetic polymers (in the case of rigid layer 2a).

The medical device 1 may include a case 1a stably engaged to the holder 2 for housing one or more electrical/electronic components of the medical device 1 itself which will be better detailed later. In the example shown in Figure 2, the case 1a may has a cylindrical or hemispherical conformation. In particular, the case 1a may define, in cooperation with the holder 2, a watertight internal volume, completely separated from an external environment and capable of preventing the passage of gases or fluids.

The medical device 1 includes at least one sensor 5 carried by the holder 2, e.g. housed in the inner volume of the case 1a, configured for monitoring a control parameter representative of a fluid of the patient P, optionally biological fluid). In the following description, the term fluid of the patient or biological fluid of the patient is referred to blood or interstitial fluid. The sensor 5 may be configured for emitting a signal representative of the control parameter, for example related to the percentage of glucose in the fluid of the patient or a percentage of glycated hemoglobin, and sending that signal to a control unit 50 detailed below.

The sensor 5 may implement one or more techniques for glucose measurement, such as: occlusion/diffusion spectroscopy, optical coherence tomography, bioimpedance spectroscopy, millimeter wave detection, microwave detection, and high-frequency wave detection. Depending on the glucose measurement technique adopted, the sensor 5 may include: an infrared sensor, a luminescence sensor, an electromagnetic radiation sensor, an acoustic sensor, an ultrasonic sensor, an impedance measurement circuit, and a radio frequency sensor.

In an embodiment, the sensor 5 is placed in contact with the skin of the patient and implements, in a non-limiting manner, a bioimpedance spectroscopy technique, which involves measuring the impedance from the patient's body to the passage of an alternating electric current at low intensity (e.g., equal to 800µA) and at a predetermined frequency. For the generation of the signal of the control parameter representative of the glucose concentration in the fluid of the patient, the aforementioned technique, uses the permittivity and conductivity properties of red blood cells; in particular, the implemented technique allows for the determination of a change in the glucose level following the detection of a change in red blood cells in the blood. The sensor 5 may have one or more electrodes placed in contact with or near the skin of the patient P configured for emitting, as mentioned above, an electric current signal to estimate the percentage of glucose present in a composition of the fluid of the patient.

The medical device 1 also includes a battery 6 carried by the holder 2, e.g. also housed in the inner volume of case 1a; the battery 6 is electrically connected to the sensor 5 and configured to power it. The battery 6 may be a rechargeable battery of the solid-state type, optionally lithium iodide, or a conventional lithium-ion battery. The battery 6 has a charging capacity comprised between 0.5 Ampere-hour and 10 Ampere-hour and is configured for supplying a continuous output voltage, for example, comprised between 2.5 Volt and 5 Volt, optionally substantially equal to 3.3 Volt.

The battery 6 is connected to the control unit 50 for its power supply; in fact, the battery 6 is used as the power supply component of the device that may supply power to both the sensor 5 and the control unit 50. The control unit 50, connected to the battery 6, is configured for estimating a value of a charging level of the battery itself. In particular, the control unit 50 is configured for performing a safety procedure comprising the steps of:
- estimating a charging level of the battery 6,
- comparing the estimated charging level of the battery 6 with a threshold value of minimum charge, for example comprised between 20% and 30% of the total electrical energy that may be stored in the battery 6,
- if the value of the charging level of the battery 6, estimated by the control unit 50, is lower than the threshold value of minimum charge, the same control unit 50 may be configured for commanding a data transmitter 15 (later detailed), for emitting an alarm signal to warn the patient of a low charging level of the battery 6.

As will be better described later, the medical device 1 may include at least one biofuel cell 3 configured for using substances in the fluid of the patient to produce electricity; this energy may be used by the device to charge the battery 6.

Thus, the medical device 1 may include an energy converter 7 carried by the holder 2, in particular the rigid layer 2a, and interposed between the biofuel cell 3 and the battery 6. In detail, the energy converter is housed in the inner volume of the case 1a. The energy converter 7 is configured for receiving electrical energy emitted from the biofuel cell 3, convert it, and supplying electrical energy to the battery 6 at a predetermined voltage for charging the battery 6 itself. For example, the energy converter 7 may include a voltage regulator connected to the battery 6 and configured to supply in output a constant electrical voltage greater than an input electrical voltage, which is also constant. The voltage regulator may be a conventional DC-DC power converter, such as a *step-up regulator,* a *buck-boost converter,* or a *Sepic converter,* having an input voltage comprised between 0.5 Volt and 5.5 Volt, and a constant output voltage of essentially 3.3 Volt. The energy converter 7, optionally the voltage regulator, is configurable between:
- an active condition wherein it supplies a constant output voltage as indicated above, and
- a deactivated condition wherein it does not supply output voltage.

The control unit 50 may be electrically connected to the energy converter 7 and configured for:
- determining an input voltage value to the energy converter 7 itself (equivalent to a voltage value measured at the ends of the biofuel cell 3),
- comparing the input voltage to energy converter 7 with an electrical threshold value, optionally comprised between 0.2 Volt and 0.4 Volt,
   ∘ in case the input voltage is higher than the electrical threshold value, the control unit 50 is configured for commanding the active condition of the energy converter 7 and allowing electricity to be supplies to the battery 6,
   ∘ in case the input voltage is lower than the electrical threshold value, the control unit 50 is configured for commanding the deactivated condition of the energy converter 7.

In one variant, the transition between the active and deactivated condition of the energy converter 7 may be internally managed by the converter itself depending on a value of the input voltage; in such a variant, it is not the control unit 50 that controls the transition between the active and deactivated condition but this is managed directly by the energy converter through dedicated circuitry implementing digital or analog logic. If the input voltage to the energy converter 7 is lower than the electrical threshold value, the voltage regulator remains in the deactivated condition, while the energy converter 7 switches its state from the deactivated condition to the active condition if the input voltage is within or exceeds the electrical threshold value (optionally defined between 0.2 Volt and 0.4 Volt).

The energy converter 7 may also include a temperature sensor configured for generating a signal representative of the temperature of the external environment surrounding the energy converter 7 (optionally the voltage regulator). The energy converter 7 may have a circuit component suitable for receiving the temperature signal sent by the temperature sensor and estimating a respective temperature value. The circuit component, depending on the estimated temperature value, is configured for controlling the transition between the active and deactivated condition of the energy converter 7 if the estimated temperature value exceeds a predetermined temperature range, optionally comprised between 39°C and 50°C. This feature of the energy converter 7 ensures its operation in prefixed temperature range, thus limiting overload or overtemperature conditions that may cause malfunctions or failures of the medical device 1. It should also be noted how the above temperature values may be variable in the specified range depending on the extent of the skin surface of the patient.

As mentioned above, the medical device 1 includes a biofuel cell 3 carried by the holder 2, e.g., also arranged in the inner volume of the case 1a; the biofuel cell 3 is configured for producing electrical energy, e.g., intended for charging the battery 6. The biofuel cell 3 is electrically connected to the energy converter 7 for charging the battery 6 and configured to generate an output voltage signal directed to the energy converter 7: the input voltage to energy converter 7 coincides with the output voltage from biofuel cell 3.

The biofuel cell 3 is configured for producing electrical energy through the chemical transformation of the biological fluid of the patient (as mentioned above through the treatment of the blood and/or interstitial fluid of the patient). In an example, the biofuel cell 3 may be an enzymatic cell suitable for performing chemical transformation to obtain electrical energy, such as using enzymes as a catalyst to oxidize its fuel. The enzymatic biofuel cell is suitable for using at least one of the following enzymes to produce electricity: glucose oxidase (GOx), bilirubin oxidase (BOD), laccase, pyrroloquinoline quinone (PQQ), and glucose dehydrogenase (GDH), which ensure the oxidation of various monosaccharides and disaccharides, e.g., glucose, maltose, lactose, galactose, xylose, and mannose.

The biofuel cell 3 has a case wherein one or more chambers are defined for the passage of a biological fluid of the patient (e.g., blood), each of which is placed in fluid communication with one or more successively detailed needles or micro-needles, which are in charge of withdrawing and re-injecting the blood into the patient. The biofuel cell 3 also has a first and second electrode (anode and cathode), housed in a chamber of the case in contact with the blood and configured for reacting with the latter for producing energy. The first and second electrodes have a platelike conformation (e.g., square or rectangular), having respective inner surfaces mutually facing each other and respective outer surfaces, opposite the inner surfaces, configured for contacting the blood. The first electrode has a selective membrane, e.g., glucose selective, carried by the outer surface and configured for contacting the biological fluid, while the second electrode has an oxygen selective membrane carried by the respective outer surface of the second electrode, also configured for contacting the biological fluid. The biofuel cell 3 also includes a hydrogen ion selective membrane (H⁺) defined in interposition between the respective inner surfaces of the first and second electrodes. From the materials point of view, each electrode may have either a carbon structure or a carbon nanotube (CNT) structure, such as made of Buckypaper and coated with one of the following enzymes: glucose oxidase (GOx), bilirubin oxidase (BOD), laccase, pyrroloquinoline quinone (PQQ) and glucose dehydrogenase (GDH). The respective carbon nanostructures of the first and second electrodes may be respectively coated with pyrroloquinoline quinone-glucose dehydrogenase (PQQ-GDH) and laccase.

The aforementioned enzymes allow the first and second electrodes to produce electrical energy through the oxidation of a substance in the biological fluid, for example, the oxidation of glucose to gluconolactone, and the reduction of oxygen to water. These reactions generate a potential difference at the ends of the first and second electrodes (open circuit potential), which is proportional to the voltage signal output from the biofuel cell 3.

As mentioned, the medical device 1 may also comprise a first and second needle 11, 12, carried by the holder 2, optionally by the rigid layer 2a, and crossing the adhesive layer 2b (Figure 2) to fit under the skin of the patient. For example, the first and second needles 11, 12 may be in the form of micro-needles having respective channels for the passage of biological fluid, each having a diameter comprised between 5 µm and 50 µm. The first and second needles 11, 12 are configured for allowing biological fluid to be withdrawn from the patient, allow it to pass through the biofuel cell 3, and feed the fluid back into the patient. In terms of materials, the first and second needles 11, 12 may both be made of a glassy carbon structure or single-crystalline silicon.

In detail, the first needle 11 comprises a plurality of micro-needles each of which has a length comprised between 100 and 500 µm, optionally a diameter comprised between 5 and 50 µm; in greater detail, said at least one first needle 11 has a number of micro-needles greater than 50, optionally between 100 and 5000, distributed on the holder 2, optionally uniformly, with a density greater than 50 micro-needles per cm². Similarly, the second needle 12 comprises a plurality of micro-needles each of which has a length comprised between 100 and 500 µm, optionally a diameter comprised between 5 and 50 µm; in greater detail, said at least one second needle 12 has a number of micro-needles greater than 50, optionally comprised between 100 and 5000, distributed on holder 2, optionally uniformly, with a density greater than 50 micro-needles per cm².

The plurality of micro-needles of the first and second needles are carried by the holder and emerging from the adhesive layer; in detail, the plurality of micro-needles crosses the thickness of the holder such that they carry the fluid of the patient to the components of the medical device designed to receive said fluid.

The use of a plurality of micro-needles rather than a single needle in inlet and a single needle in outlet, may allow the withdrawal of interstitial fluid from different areas of adipose tissue, increasing the accuracy of blood glucose measurements and reducing the variability of results. Furthermore, since the plurality of micro-needles defines a larger fluid withdrawal area than the area covered by a single needle; thus, the flow of interstitial fluid is more evenly distributed than with a single needle, preventing the formation of pockets of stagnant interstitial fluid that could compromise the accuracy of measurements. Additionally, the presence of a plurality of micro-needles allows interstitial fluid to be withdrawn continuously and with a constant flow, as opposed to a single needle in inlet and only one needle in outlet, which may cause unstable and variable flow. This ensures a constant supply of glucose to the biofuel cell, enabling constant power generation. Although the presence of micro-needles allows for several advantages, the use of at least one needle (11, 12) also allows the capabilities of the medical device described above to be exploited for the production of electricity and thus the charging of the device itself.

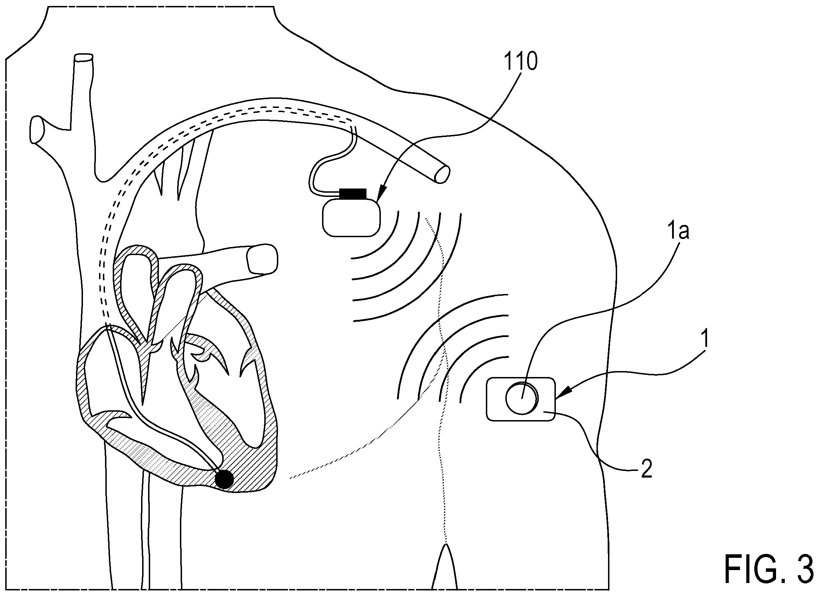

The medical device 1 includes a pump 4 carried by the holder 2, in particular by the top layer 2a of the holder, and housed in the inner volume of the case 1a. As, for example, shown in Figure 3, the pump 4 is in fluid communication with said first and second needles 11, 12 and with the biofuel cell 3; the pump 4 is configured to allow fluid, e.g., blood, to be withdrawn from the patient's biological environment via the first needle 11, sent to biofuel cell 3 and then returned to the patient's biological environment via the second needle 12. In fact, each needle is placed in fluid communication with the pump 4, through which the biological fluid is passed through the biofuel cell 3: the first needle 11 is configured for withdrawing biological fluid and supplying it to the pump 4, while the second needle 12 is in fluid communication with the biofuel cell 3 and is configured for receiving the biological fluid passing through the cell and feeding it back into the patient.

The pump 4 is electrically powered by the battery 6 and configurable at least between:
- an activation condition wherein the pump 4 moves the biological fluid of the patient (e.g., blood) from the first needle 11, through the biofuel cell 3 and finally out of the second needle 12, and
- an inactive condition wherein it does not allow biological fluid to pass through the biofuel cell 3.

The pump 4 may also include at least one electric motor, optionally supplied with DC voltage (optionally at a voltage corresponding to the voltage deliverable by the battery 6 essentially equal to 3.3 Volt). The control unit 50 is active in command on the electric motor to control the activation/inactive condition of the pump.

As mentioned, the medical device 1 may comprise the control unit 50 carried by the holder 2, in particular the top layer 2a, and placed in the inner volume of the case; the control unit 50 is configured for operating in command over one or more of the above components. The control unit 50 may include a digital microprocessor that can be powered by the battery 6 and a memory for storing data, such as the threshold value of the control parameter, the electrical threshold value, and the threshold value of minimum charge of the battery 6. The memory may be a non-rewritable type, such as a ROM memory wherein predefined data are stored, or the memory may be a rewritable memory, such as a solid-state type, wherein editable values may be stored, such as by means of a data-entry unit (not shown in the accompanying figures).

The control unit 50 is connected to the sensor 5 and activate in control on the pump 4, which is configured to perform an electricity production procedure for charging the battery 6 during which the control unit 50 commands the activation of the pump 4 to produce electricity to be supplied to the battery 6. In detail, the power production procedure may include the following steps:
- receiving one or more signals from the sensor 5, representative of the control parameter of the biological fluid, e.g., blood and/or interstitial fluid, of the patient,
- processing each signal emitted by the sensor 5 to estimate respective values of the control parameter,
- comparing each estimated value of the control parameter with a threshold value, and
- based on said comparison, commanding the activation of pump 4 for supplying a predetermined amount of the biological fluid of the patient, e.g., blood and/or interstitial fluid, to the biofuel cell 3 for power generation.

In detail, the control parameter may include the glycemic rate in the blood of the patient. In this condition, the comparison step of the power generation procedure involves comparing the estimated value of glucose in the blood of the patient with the threshold value (optionally comprised between 80 mg/dl and 100 mg/dl). The control unit 50 is then configured for commanding the activation of the pump 4 in the event that the estimated glucose value exceeds the aforementioned range so that the oxidation of glucose allows the biofuel cell 3 to produce electricity to be supplied to the battery for recharging and, at the same time, for reducing the glycemic rate in the blood of the patient.

During the activated condition of the pump 4, the control unit 50 is configured for continuously receiving signals from the sensor 5, representative of the control parameter for continuous estimation of the glucose value in the fluid of the patient; each estimated glucose value is compared with the threshold value so that the control unit may keep the pump 4 in the activated condition only when the glucose value is higher than the threshold value.

For example, the control unit 50, once the presence of a glucose value in the fluid of the patient above the threshold value has been established, may be configured for calculating and treating an amount of fluid sufficient to reestablish the values of glucose of the fluid at or below the threshold value. The control unit 50 is then configured for commanding the activation of the pump 4 for a predetermined time interval wherein it supplies the previously calculated amount of fluid to the biofuel cell 3 for generating electric energy. The amount of fluid may be a function of a flow rate of fluid deliverable by the pump 4: thus, the medical device 1 may include a flow rate sensor connected to the control unit 50 and configured for emitting a signal representative of a flow rate of fluid of the patient passing through the pump 4. The power generation procedure may further comprise the steps of receiving the signal emitted by the flow rate sensor, based on said signal estimating the flow rate of fluid being delivered by the pump 4, and consequently commanding the activation of the pump 4 for the predetermined period of time during which the amount of fluid of the patient is delivered to the biofuel cell 3.

Thus, the control unit 50 allows for using hyperglycemic conditions in the fluid of the patient for the production of electricity via the biofuel cell 3 for the subsequent recharging of the battery 6; in this way, it is possible to recharge the medical device 1 and keep the fluid glycemic rate in the patient under control so as to avoid hyperglycemic conditions. The medical device 1 may include a data transmitter 15 carried by the holder 2, optionally by the rigid layer 2a and housed in case 1a. The data transmitter 15 is configured for transmitting at least one data signal to an external mobile device 120, such as a smartphone, tablet, or computer. The data transmitter 15 is connected to the control unit 50 and configured for commanding the activation of the data transmitter 15, for allowing a monitoring signal to be sent to the external mobile device 120 including at least the values of the control parameter. In particular, the control unit 50 is active in command on data transmitter 15 and is configured for performing a monitoring procedure comprising the following steps:
- receiving one or more signals from the sensor 5,
- process each signal emitted by the sensor 5 and estimating respective values of the control parameter of the fluid,
- comparing each estimated value of the control parameter with a threshold value, and
- commanding the activation of the data transmitter 15 to emit a monitoring signal to the external mobile device 120 representative of the values of the control parameter of the fluid of the patient.

Therefore, the monitoring procedure allows the patient to view, via the external mobile device 120, the blood glucose values detected by the sensor 5.

The control unit 50 may also be configured for detecting a malfunctioning condition of the medical device 1, which may be determined, for example, according to the temperature value detected by the temperature sensor in the energy converter 7, for example, if it exceeds a predetermined threshold value. The control unit 50, if it detects the malfunction condition, is configured for commanding the data transmitter 15 to send a respective signal to the external mobile device 120 for notifying the patient of a malfunction of the medical device 1.

The control unit 50 is configured for commanding the data transmitter 15 to issue an alarm signal when it determines a value of the charging level of the battery 6 lower than the threshold value of minimum charge. The control unit 50, through the data transmitter 15, allows the patient to be alerted of a low charging level of the battery. The data transmitter 15 is also configured for notifying the patient if a malfunctioning condition of the medical device 1 is detected, or to send the monitoring signal representative of the blood glucose values detected by the sensor 5. The data transmitter 15 may include a wireless data transmission module, such as one that may take advantage of Bluetooth, Wi-Fi or infrared technology.

In a variant not shown in the accompanying figures, the medical device 1 may be without the energy converter 7 and configured to decrease or modulate the percentage of glucose in the blood of the patient without recharging the battery 6 of the medical device 1 itself. In the latter configuration, the medical device 1 is configured, as previously described, to compare the estimated values of the control parameter with the value of a glucose threshold parameter in the fluid of the patient, for example, comprised between 60 mg/dl and 100 mg/dl. Depending on the outcome of said comparison, the control unit 50 may be configured for commanding the activation of the pump 4 to supply the predetermined amount of fluid of the patient to the biofuel cell 3 and allow oxidation of the glucose in the fluid of the patient.

The medical device 1 may include a charge receiver 16 (Figure 3) directly connected to the battery 6 or connected to battery 6 via the energy converter 7; the charge receiver 16 is configured to receive energy from a source external to the medical device 1 and generating electrical energy for charging the battery 6. The charge receiver 16 may, for example, comprise an antenna carried by the holder 2, optionally housed in the inner volume of the case, configured for receiving electromagnetic waves, e.g., microwaves, to generate electrical voltage useful for charging the battery 6. For example, the antenna may be a coil or winding suitable for generating an alternating electric current that, when converted to direct current, is used for charging the battery 6. The alternating electric current generated by the antenna may be sent to the energy converter 7 or an AC-DC converter, and then sent to the battery 6.

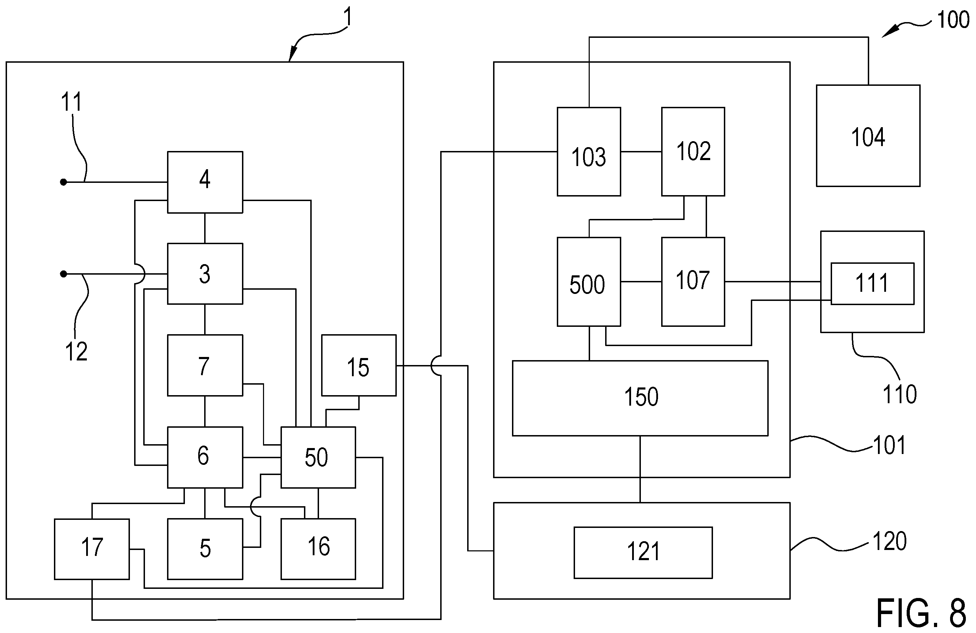

As shown in Figure 8, the medical device 1 may also include a charge transmitter 17 connected to the battery 6 and configured for emitting electromagnetic waves employable for charging an additional device unrelated to the medical device 1, such as an implantable device 101 present in the patient. The charge transmitter 17 may include an antenna carried by the holder 2, optionally housed in the inner volume of the case, configured for generating electromagnetic waves, optionally microwaves, for charging the implantable device 101. For example, the antenna may be a coil or winding suitable for generating a magnetic field configured for inducing in the implantable device 101, an alternating electric current usable for charging the implantable device 101 itself. The charge transmitter 17 is connected to the control unit 50 and configured for commanding it, based on a charging level of the battery 6, to emit electromagnetic waves for charging the implantable device 101. As previously mentioned, the control unit 50 is configured for detecting a charging level of the battery 6 and comparing it with the threshold value of minimum charge (e.g., comprised between 20% and 30% of the total storable energy in the battery 6). If the charging level is higher than the threshold value of minimum charge, the control unit 50 is configured for commanding the charge transmitter 17 to emit electromagnetic waves to charge the implantable device 101; vice versa, the control unit 50 is configured for commanding, via the data transmitter 15, to emit an alarm signal to notify the patient of a low charging level of the battery 6. The operation of the medical device in cooperation with the implantable device will be described in detail later in connection with the description of a medical system.

The medical device 1 is employable to treat subjects with diabetes or subjects without any particular disease who wish to keep certain blood parameters, such as glycemic rate, under control. Thus, the medical device 1 is employable to perform therapeutic treatment methods; however, it may also be used by healthy subjects for mere monitoring of blood parameters, without performing any therapeutic treatment.

### Recharging method of the medical device

A method of recharging performed by the medical device 1 above and/or according to the accompanying claims is also described.

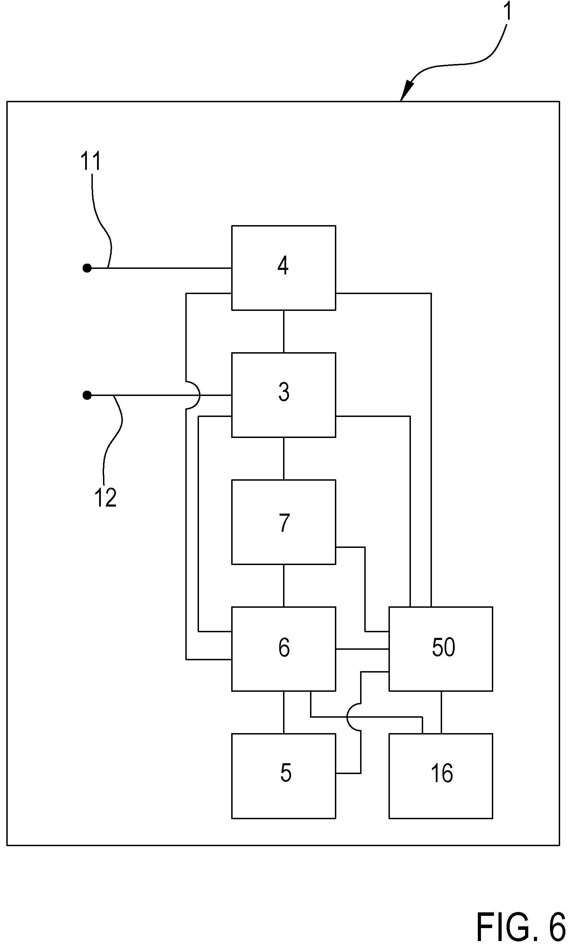

As shown in Figure 6, the recharging method involves the steps of detecting, using the sensor 5, one or more signals representative of a control parameter of the fluid of the patient (e.g., blood and/or interstitial fluid), and estimating respective values of the control parameter of the fluid (step 200). The method may also include a step of comparing each estimated value of the control parameter with the threshold value, the latter being comprised between 60 mg/dl and 100 mg/dl (step 202). If results from the comparison in step 202 that the estimated value of the control parameter is lower than the threshold value, the method involves detecting additional values of the control parameter using the sensor 5 (see the return line indicated by the reference number 201). If, on the other hand, the comparison in step 202 results in the estimated value of the control parameter being greater than the threshold value, the method involves commanding the activation condition of the pump 4 (step 203) to allow the biological fluid of the patient to pass through the biofuel cell and consequently to allow energy production.

The method also includes a step of estimating a value of an electrical parameter (e.g., a voltage signal output from the biofuel cell) representative of the electrical energy produced by the biofuel cell (step 204) and then, comparing it with an electrical threshold value comprised between 0.2 Volt and 0.4 Volt (step 205). If the electrical parameter value is equal to or greater than the electrical threshold value, the method involves a step of supplying the energy produced by the biofuel cell to the battery 6 to recharge it (step 207). If the value of the electrical parameter is lower than the electrical threshold value, the method involves detecting additional values of the control parameter using the sensor 5 (see the return line indicated by the reference number 206) and interrupting the transit of the voltage signal output from the biofuel cell 3.

The method also involves to cyclically performing the above steps 200-207 until the battery 6 has a sufficiently high state of charge to allow proper power supply to each electrical/electronic element of the medical device.

The method may also involve performing one or more steps to detect and determine the charging level of the battery 6; specifically, the method involves:
- detecting a charging level of the battery 6,
- comparing the charging level of the battery with a threshold value of minimum charge (e.g., comprised between 20% and 30% of the total storable energy in the battery 6), and
- if the charging level is lower than the threshold value of minimum charge, emitting an alarm signal (via the data transmitter 15) to notify the user of a low level of remaining charge of the battery 6.

The method may also include a charging phase of the battery 6 by recurring to the use of an external charging source, such as a wall charger that may be physically connected to the battery 6 via a miniUSB, microUSB, or USB-C connector, or via an inductive charging base.

As mentioned above, the recharging method may be a method that can be performed by healthy subjects for the mere monitoring of parameters in the blood. However, the method may also be a therapeutic treatment method when used by subjects with diabetes and performed for the purpose of maintaining blood glucose levels below predetermined threshold values.

### Implantable device

An implantable device 101 that may be used to charge an implantable electronic component 110 such as a pacemaker, which may also be installed under the skin of the patient and configured for performing predetermined functions, is also described.

The implantable device 101 may be a module independent from the implantable electronic component 110, which may be installed in the biological environment of the patient, such as in a subclavicular or abdominal area, suitable for interacting with the implantable electronic component itself. The implantable device 101 is electrically and/or communicatively connectable to the implantable electronic component 110, such as by means of an electrical cable. The implantable device 101 includes a case 101a suitable for housing one or more electrical/electronic components. The case is of the watertight type, suitable for isolating each component from the biological environment of the patient and preventing the passage of fluids or gases such as to jeopardize the proper functioning of the implantable device itself.

As shown in Figure 8, the implantable device 101 includes a battery 102 housed in the case 101a and configured for powering each of the electrical/electronic elements of the implantable device 101. The battery 102 may be a rechargeable solid-state type battery, optionally lithium iodide, or a conventional lithium-ion type battery. The battery 102 has a charging capacity comprised between 2 Ampere-hour and 10 Ampere-hour and is configured for supplying a continuous output voltage of essentially 3.3 Volt. The battery 102, besides of enabling the electrical/electronic elements of the implantable device 101 to be powered, also allows energy to be stored for charging the implantable electronic component 110 as detailed in the following.

The implantable device 101 may include a control unit 500 connected to the battery 102 and configured to receive a signal representative of a charging level of the same battery 102. The control unit 500 is also configured to estimate a value of the charging level of the battery 102 and compare it with a threshold value of minimum charge, for example, comprised between 20% and 30% of the total storable energy in the battery 102. If the value of the charging level is lower than the threshold value of minimum charge, the control unit 500 is configured to notify the patient of a low charging level of battery 102 and request recharging.

The implantable device 101 may also include a charge receiver 103 connected to the battery 102 of the implantable device 101, configured to receive power from an external power source, such as from the medical device 1 described above or from an external charging source 104, for charging the battery 102. The charge receiver 103 of the implantable device 101 may, for example, be an antenna housed in the case 101a, configured to receive electromagnetic waves, such as microwaves, to generate an electrical voltage. For example, the antenna may be a coil or winding suitable for generating an alternating electric current that, when converted to direct current, is used to charge the battery 102. The implantable device 101 may also include a charge transmitter 107 housed in the case 101a of the implantable device 101 and configured for allowing charging of implantable electronic component 110. The charging transmitter 107 may be a conventional DC-DC power converter, such as a *step-up regulator, buck-boost converter,* or *Sepic converter,* having an input voltage comprised between 0.5 Volt and 5.5 Volt, and a constant output voltage of 3.3 Volt. The implantable device 101 may include a data communication module 150 housed in the case 101a of the implantable device, configured for transmitting data to an external mobile device 120 such as a smartphone, tablet, or computer. The data communication module 150 is connected to the control unit 500 of the implantable device 101, configured to send an alarm signal to the patient to warn the patient itself of a malfunction of the implantable device or a low remaining charge of the battery 102 of the implantable device itself. The control unit 500, if it detects a low charging level of the battery 102 of the implantable device, such as when the value of the charging level of the battery 102 is lower than the threshold value of minimum charge, is configured for commanding the data communication module 150 to emit the alarm signal. The data communication module 150 may include a wireless data transmission module, such as using Bluetooth, Wi-Fi, or infrared technology.

As anticipated, the implantable device 101 may has a control unit 500, housed in the case 101a and connected to battery 102, the charge transmitter 107 and the data communication module 150. The control unit 500 of the implantable device 101 may implement fully analog logic, or include a microprocessor implementing a digital logic. In one variation, the implantable device 101 may be integrated into the implantable electronic component 110.

### Medical system

A medical system 100, comprising an implantable electronic component 110 that may be installed under the skin and of a patient and configured for performing predetermined functions, is also described. In an example, the implantable electronic component 110 may be a pacemaker pre-positioned to electrically stimulate the contraction of the heart of the patient, or a cardiac defibrillator, a sacral nerve stimulator, or a glucose meter. The implantable electronic component 110 may have an electric energy accumulator 111 for power supplying one or more of the electrical/electronic components of the implantable electronic component itself. The electric energy accumulator 111 may, for example, be a rechargeable battery of the solid-state type, optionally lithium iodide type, or a conventional lithium-ion battery.

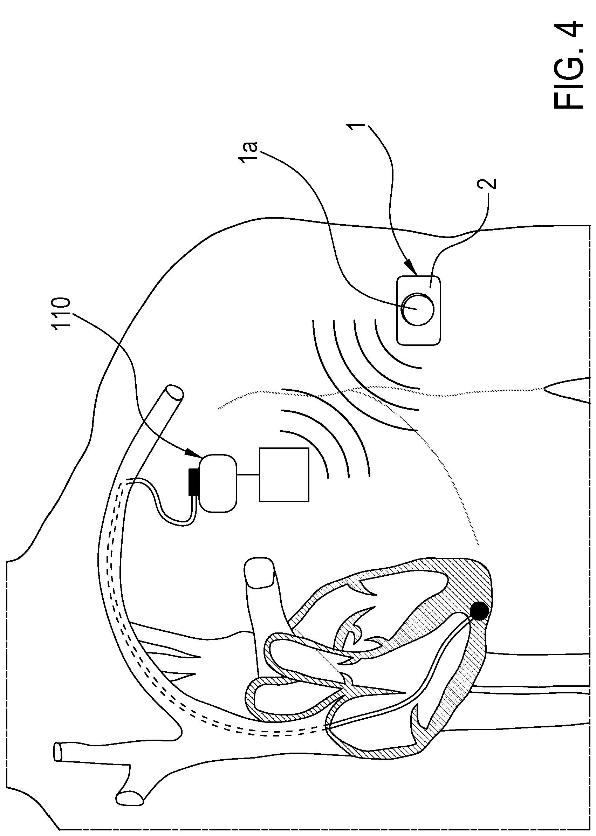

The system may also include the implantable device 101 described above which is suitable for interacting with the implantable electronic component, for example, for allowing its recharging. In the example of Figure 4, the implantable device 101 is an independent module from the implantable electronic component 110 that may be installed in the biological environment of the patient, such as in a subclavicular or abdominal area, which is suitable for interacting with the implantable electronic component itself. The implantable device 101 is electrically and/or communicatively connected to the implantable electronic component 110, such as by an electrical cable.

The medical system 100 also includes a charging source 104 configured to transmit electrical energy to the charge receiver 103 of the implantable device 101 for recharging of the battery 102 of the implantable device. The charging source 104 includes a transmitting antenna configured for generating electromagnetic waves, optionally microwaves, detectable by the charge receiver 103 for generating electrical energy. For example, the charging source 104 may comprise a wireless charging base, external to the patient. The charging source 104 may, in addition to or as an alternative to the charging base, comprise at least one medical device 1 according to the preceding description. To allow efficient energy transfer, it is preferable to place the charging source 104 in close proximity to the charge receiver 103 of the implantable device 101, particularly at a distance comprised between 5 mm and 30 mm. In case the charging source has one or more electrical windings, these may be superimposed on the electrical windings of the charge receiver 103 of the implantable device 101.

As mentioned above, the implantable device 101 may include a charge transmitter 107 housed in the case 101a of the implantable device 101 and configured for charging the implantable electronic component 110. The charge transmitter 107 is connected to the battery 102 of the implantable device 101 and also connected to the implantable electronic component 110 to recharge the electric energy accumulator 111. The charging transmitter 107 may be a conventional DC-DC power converter, such as a *step-up regulator, buck-boost converter or Sepic converter,* having an input voltage comprised between 0.5 Volt and 5.5 Volt, and a constant output voltage of 3.3 Volt.

As mentioned above, the implantable device 101 may include a data communication module 150; in an embodiment of the implantable device 101, the data communication module 150 is also configured to communicate with the medical device 1 (when the charging source 104 includes said medical device 1) to send it an alarm signal representative of a low charging level of the battery 102 of the same implantable device. The control unit 50 of the medical device 1 is configured to receive such signal and command its charge transmitter to supply energy to the implantable device for charging the battery 102 of the implantable device. Note that the control unit 50 of the medical device, before commanding to supply energy to the implantable device 101, checks the remaining charging level of the battery 6. In an example, following receipt of the alarm signal from the implantable device, the control unit 50 of the medical device 1, estimates the charging level of its battery 6 and compares it with the threshold value of minimum charge. If the remaining charging level of the battery 6 of the medical device 1 is higher than the threshold value of minimum charge, the control unit 50 of the medical device 1 commands to supply energy via the charge transmitter 17 of the medical device 1 to recharge the energy accumulator 111 of the implantable electronic component 110.

As anticipated, the implantable device 101 may have a control unit 500; the control unit 500 is also connected to the implantable electronic component 110 and in particular to the electric energy accumulator 111, configured to estimate a residual charging level of the same electric energy accumulator 111. The control unit 500 is also configured to compare the estimate of the residual charging level with the threshold value of minimum charge. If a low residual charging level of the energy accumulator is determined, such as if the residual charge estimate is lower than the threshold value of minimum charge, the control unit 500 is configured to command the transfer of energy from the battery 102 to the electric energy accumulator 111 of the implantable electronic component, allowing it to be recharged. The medical system 100 may include a mobile device 120 that may be connected to the implantable device 101 and configured to display data sent by the latter. For example, the mobile device may be a smartphone, tablet, or computer, having a screen 121 for allowing the patient to view the alarm signal sent by the data communication module 150. The mobile device 120 may also be connected to the medical device, configured to receive the alarm signal issued by the medical device itself and notify the patient of a low level of the battery 6 of the medical device.

In a variant shown in Figure 3, implantable device 101 is integrated into the implantable electronic component 110. In such a configuration, the implantable electronic component 110 comprises a single battery, which replaces the previously mentioned battery 102 and the electric energy accumulator 111, while keeping unchanged the operation modes of the medical system described above.

### Charging method of the medical system

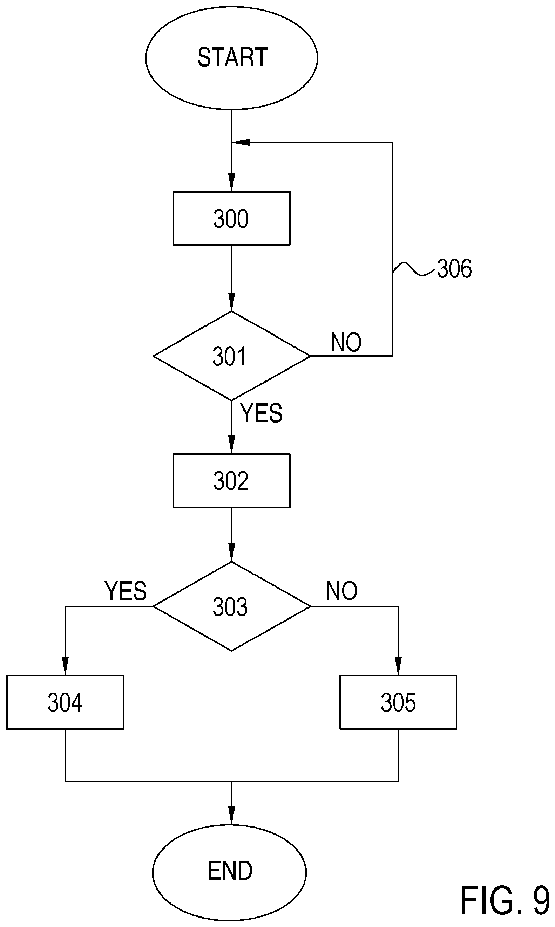

A method for charging the medical system 100 is also described. As shown in Figure 9, the method involves a first step of estimating one or more values of a residual charging level of the electric energy accumulator 111 of the implantable electronic component (step 300) and comparing each estimated value of the charging level of the electric energy accumulator 111 with a respective threshold value of minimum charge comprised between 20% and 30% of a total accumulable energy of the electric energy accumulator 111 (step 301). In case the above comparison indicates an estimated value of the charging level higher than the threshold value of minimum charge of the energy accumulator, the method involves re-performing step 300 (see recirculation line 306 in Figure 9). In case the above comparison indicates an estimated value of the charging level lower than the threshold value of minimum charge of the electric energy accumulator 111, the method involves estimating one or more values of the charging levels of the battery 102 of the implantable device (step 302) and comparing them with the threshold value of minimum charge of the battery 102 (step 303). In case the residual charge level of the battery 102 of the implantable device is higher than the threshold value of minimum charge, for example, comprised between 20% and 30% of a total accumulated energy from the battery 102 of the implantable device 101, the method involves a step of commanding the implantable device 101 to supply, via the charge transmitter 107, electrical energy accumulated in the battery 102 to the electrical energy storage 111 (step 304). In case that the residual charging level of the battery 102 is lower than the threshold value of minimum charge, the method involves sending an alarm signal, via the data communication module 150, to the mobile device 120 to notify the patient of a low residual charging level of both the battery 102 of the implantable device 101 and the electric energy accumulator 111 of the implantable electronic component 110 (step 305).

The charging of the electrical energy storage 111 of the implantable electronic component is performed by placing the charging source 104 close to the implantable device 101, for transferring energy between the two devices. This step involves placing the charging source 104 in close proximity to the charge receiver 103 of the implantable device, such as at a distance comprised between 5 mm and 30 mm.

Thus, the method involves a step of receiving and storing energy accumulated by the battery 102 of the implantable device 101 via the charge receiver 103 and transferring energy, via the charge transmitter 107 of the implantable device, to the electric energy accumulator 111 of the implantable electronic component 110. In a variant, if the residual charging levels of the battery 102 of the implantable device 101 and the electric energy accumulator 111 are lower than respective threshold value of minimum charge, the method includes a step of transmitting an alarm signal to the medical device 1 via the data communication module 150. Following receipt the alarm signal via a data receiver of the medical device, the method includes a step of receiving and estimating one or more values of the charging level of the battery 6 of the medical device 1 and comparing them with a threshold value of minimum charge of the medical device 1, for example, comprised between 20% and 30% of a total energy storable by said battery 6 of the medical device 1. In the event that the value of the charging level of the battery 6 is higher than the threshold value of minimum charge, the method includes commanding to the medical device 1, the transfer of energy, via its own charge transmitter 17, from the battery 6 of the medical device 1 to the battery 102 of the implantable device 101. Subsequently, the method involves transferring energy from the battery 102 of the implantable device 101 to the electrical energy storage 111 of the implantable electronic component 110 via the charge transmitter 107 of the implantable device 101.

## Claims

1. Medical device (1) comprising:
- a holder (2) applicable on the skin of a patient (P),
- at least one rechargeable battery (6) carried by the holder (2),
- at least one biofuel cell (3) carried by the holder (2) and connected to the battery (6), wherein the biofuel cell (3) is configured for receiving a fluid from the patient (P) and producing electrical energy for recharging the battery (6), **characterized by**
- at least one pump (4) carried by the holder (2) and configured for supplying the fluid of the patient to the biofuel cell (3),
- at least one sensor (5) carried by the holder (2) and configured for emitting a signal representative of a control parameter of the fluid of the patient,
- a control unit (50) connected to the pump (4) and the sensor (5) and active in control on said pump (4), wherein the control unit (50) is configured for performing a power generation procedure comprising the following steps:
∘ receiving a signal from the sensor (5),
∘ processing the signal emitted by the sensor (5) and estimating a value of the control parameter of the fluid of the patient,
∘ comparing the estimated value of the control parameter with a threshold value, and
∘ based on said comparison, commanding the activation of the pump (4) for supplying the fluid of the patient to the biofuel cell (3) for producing electricity.

2. Medical device according to claim 1, wherein said control parameter includes at least one of: blood glucose, glycated hemoglobin, oxygen concentration, heart rate, blood pressure, temperature, amino acid or fat concentration.

3. Medical device according to any one of the preceding claims, wherein the sensor (5) is an optical glucose sensor, optionally the sensor (5) includes at least one selected in the group of: an infrared sensor, a luminescence sensor, an electromagnetic radiation sensor, an acoustic sensor, an ultrasonic sensor, an impedance measurement circuit, a radio frequency sensor.

4. Medical device according to any one of the preceding claims, wherein the control unit (50), during the estimation step of the value of the control parameter of the power generation procedure, is configured for estimating the value of glucose in the fluid of the patient,
wherein the control unit (50), during the subsequent comparison step of the power generation procedure, is configured for comparing said fluid glucose value with a threshold value, optionally said threshold value is comprised between 60 mg/dl and 100 mg/dl.

5. Medical device according to the preceding claim, wherein the activation step of the pump (4) of the power generation procedure is performed in case the control unit (50) determines an estimated glucose value greater than the threshold value.

6. Medical device according to any one of the preceding claims, wherein the control unit (50) is connected to the biofuel cell (3) which is configured for emitting a signal representative of an electrical parameter representative of the electrical energy produced,
wherein the power generation procedure, performed by the control unit (50), also includes the following steps:
- receiving the signal from the biofuel cell (3),
- based on the signal emitted by the biofuel cell (3), estimating a value of the electrical parameter,
- comparing the value of the electrical parameter with an electrical threshold value,
- based on said comparison, commanding the transmission of the electrical energy produced by the biofuel cell (3) to the battery (6),
wherein the control unit (50) is configured for commanding the transmission of electrical energy produced by the biofuel cell (3) to the battery (6) when the value of the electrical parameter is greater than the electrical threshold value.

7. Medical device according to the preceding claim, wherein the electrical parameter includes at least one of: an electrical voltage measured at the ends of the biofuel cell, an electrical current, an electrical energy, an electrical resistance.

8. Medical device according to any one of the preceding claims, wherein the battery (6) is electrically connected to the pump (4), the sensor (5) and the control unit (50), wherein the battery (6) is configured for providing electrical energy to power the pump (4), the sensor (5) and the control unit (50).

9. Medical device according to any one of the preceding claims comprising an energy converter (7) interposed between the battery (6) and the biofuel cell (3), wherein said energy converter (7) is configured for receiving in input electrical energy produced by the biofuel cell (3) and emitting in output a predetermined electrical energy for charging the battery (6), optionally the output electrical energy from the energy converter is greater than the input electrical energy,
wherein the energy converter (7) includes a voltage regulator configured for receiving as input an electric voltage generated by the biofuel cell (3) and emitting in output a predetermined electric voltage, optionally comprised between 2 V and 5.5 V, for charging the battery (6), optionally the output electric voltage from the voltage regulator is higher than the input electric voltage.

10. Medical device according to any one of the preceding claims comprising a first and second needle (11, 12) suitable for allowing the passage of fluid of the patient,
wherein the first needle (11) is in fluid communication, optionally directly, with the pump (4) and is configured for drawing fluid from the patient and allow it to be sent to the pump (4),
wherein the second needle (12) is in fluid communication, optionally directly, with the biofuel cell (3) and is configured for receiving incoming fluid from the biofuel cell (3) and feeding it back into the patient.

11. Medical device according to any one of the preceding claims, wherein the first and second needles (11, 12) are micro-needles configured for allowing the passage of blood and/or interstitial fluid of a patient.

12. Medical device according to the preceding claim, wherein said at least one first needle (11) has a number of micro-needles greater than 50, optionally comprised between 100 and 5000, optionally each micro-needle of said at least one first needle has a length comprised between 100 and 500µ m and/or a diameter comprised between 5 and 50 m, µ
wherein said at least one second needle (12) has a number of micro-needles greater than 50, optionally comprised between 100 and 5000, optionally each micro-needle of said at least one second needle has a length comprised between 100 and 500µ m and/or a diameter comprised between 5 and 50µ m.

13. Medical device according to claim 11 or 12, wherein the holder (2) comprises at least one plaster having at least one adhesive layer (2b) defining a contact surface of the holder (2) suitable for directly contacting the skin of a patient, wherein the holder (2) extends in thickness between the contact surface and an opposing holder surface,
wherein the holder (2), optionally the plaster, directly carries the micro-needles of said first and second needles,
wherein the micro-needles of said at least one first needle (11) are distributed over the contact surface of the holder, optionally evenly distributed over said contact surface, with a density greater than 50 micro-needles per cm², optionally with a density comprised between 50 and 1000 micro-needles per cm²,
wherein the micro-needles of said at least one second needle (12) are distributed over the contact surface of the holder, optionally evenly distributed over said contact surface, with a density greater than 50 micro-needles per cm², optionally with a density comprised between 50 and 1000 micro-needles per cm².

14. Medical device according to any one of the preceding claims comprising a charge receiver (16) connected to the battery (6) and configured for receiving electromagnetic waves, optionally microwaves, from an external charger and generating electrical energy, due to said electromagnetic waves, said charge receiver (16) being configured for transmitting electrical energy to the battery (6) to recharge it.

15. Medical system (100) comprising:
- at least one implantable device (101) applicable under the skin of a patient (P), wherein said implantable device includes a battery (102) configured to power the implantable device itself,
- at least one charging source (104) configured for providing electrical energy to the battery (102) of the implantable device, wherein said charging source comprises at least one medical device (1) according to any one of the preceding claims.

## Patentansprüche

1. Medizinprodukt (1), umfassend:
- eine Halterung (2), die auf der Haut eines Patienten (P) angebracht werden kann,
- mindestens einen von der Halterung (2) getragenen Akku (6),
- mindestens eine von der Halterung (2) getragene und mit dem Akku (6) verbundene Biobrennstoffzelle (3), wobei die Biobrennstoffzelle (3) so konfiguriert ist, dass sie ein Fluid von dem Patienten (P) empfängt und elektrische Energie zum Aufladen des Akkus (6) erzeugt, **dadurch gekennzeichnet, dass**
- mindestens eine Pumpe (4), die von der Halterung (2) getragen und zur Versorgung der Biobrennstoffzelle (3) mit dem Fluid des Patienten konfiguriert ist,
- mindestens ein von der Halterung (2) getragener Sensor (5) so konfiguriert ist, dass er ein Signal ausgibt, das für einen Kontrollparameter des Fluids des Patienten repräsentativ ist,
- eine Steuereinheit (50), die mit der Pumpe (4) und dem Sensor (5) verbunden und zu der Steuerung der Pumpe (4) aktiv ist, wobei die Steuereinheit (50) so konfiguriert ist, dass sie ein Verfahren zur Stromerzeugung durchführt, das folgende Schritte umfasst:
∘ Empfangen eines Signals von dem Sensor (5),
∘ Verarbeiten des von dem Sensor (5) ausgesendeten Signals und Schätzen eines Wertes des Kontrollparameters des Fluids des Patienten,
∘ Vergleichen des geschätzten Wertes des Kontrollparameters mit einem Schwellenwert, und
∘ auf der Grundlage des Vergleichs, Steuern der Aktivierung der Pumpe (4) zur Versorgung der Biobrennstoffzelle (3) mit dem Fluid des Patienten zur Stromerzeugung.

2. Medizinprodukt nach Anspruch 1, wobei der Kontrollparameter mindestens einen von folgenden enthält: Blutzucker, glykiertes Hämoglobin, Sauerstoffkonzentration, Herzfrequenz, Blutdruck, Temperatur, Aminosäure- oder Fettkonzentration.

3. Medizinprodukt nach einem der vorhergehenden Ansprüche, wobei der Sensor (5) ein optischer Glukosesensor ist, optional enthält der Sensor (5) mindestens einen aus der folgenden Gruppe ausgewählten: einen Infrarotsensor, einen Lumineszenzsensor, einen elektromagnetischen Strahlungssensor, einen akustischen Sensor, einen Ultraschallsensor, eine Impedanzmessschaltung, einen Funkfrequenzsensor.

4. Medizinprodukt nach einem der vorhergehenden Ansprüche,
wobei die Steuereinheit (50) während des Schätzschritts des Wertes des Kontrollparameters des Verfahrens zur Stromerzeugung zum Schätzen des Glukosewertes in dem Fluid des Patienten konfiguriert ist,
wobei die Steuereinheit (50) während des nachfolgenden Vergleichschritts des Verfahrens zur Stromerzeugung so konfiguriert ist, dass sie den Glukosewert im Blut mit einem Schwellenwert vergleicht, wobei der Schwellenwert optional zwischen 60 mg/dl und 100 mg/dl liegt.

5. Medizinprodukt nach dem vorhergehenden Anspruch, wobei der Schritt des Aktivierens der Pumpe (4) des Verfahrens zur Stromerzeugung durchgeführt wird, falls die Steuereinheit (50) einen geschätzten Glukosewert bestimmt, der größer ist als der Schwellenwert.

6. Medizinprodukt nach einem der vorhergehenden Ansprüche, wobei die Steuereinheit (50) mit der Biobrennstoffzelle (3) verbunden ist, die so konfiguriert ist, dass sie ein Signal aussendet, das für einen elektrischen Parameter repräsentativ ist, der für die erzeugte elektrische Energie repräsentativ ist,
wobei das von der Steuereinheit (50) durchgeführte Verfahren zur Stromerzeugung auch die folgenden Schritte enthält:
- Empfangen des Signals von der Biobrennstoffzelle (3),
- auf der Grundlage des auf dem von der Biobrennstoffzelle (3) gesendeten Signals, Schätzen eines Wertes des elektrischen Parameters,
- Vergleichen des Wertes des elektrischen Parameters mit einem elektrischen Schwellenwert,
- auf der Grundlage des Vergleichs, Steuern der Übertragung der von der Biobrennstoffzelle (3) erzeugten elektrischen Energie an den Akku (6),
wobei die Steuereinheit (50) so konfiguriert ist, dass sie die Übertragung von elektrischer Energie, die von der Biobrennstoffzelle (3) erzeugt wird, an die Batterie (6) steuert, wenn der Wert des elektrischen Parameters größer ist als der elektrische Schwellenwert.

7. Medizinprodukt nach dem vorhergehenden Anspruch, wobei der elektrische Parameter mindestens eines enthält: eine an den Enden der Biobrennstoffzelle gemessene elektrische Spannung, einen elektrischen Strom, eine elektrische Energie, einen elektrischen Widerstand.

8. Medizinprodukt nach einem der vorhergehenden Ansprüche, wobei der Akku (6) elektrisch mit der Pumpe (4), dem Sensor (5) und der Steuereinheit (50) verbunden ist, wobei der Akku (6) so konfiguriert ist, dass er elektrische Energie zur Stromversorgung der Pumpe (4), des Sensors (5) und der Steuereinheit (50) bereitstellt.

9. Medizinprodukt nach einem der vorhergehenden Ansprüche, umfassend einen zwischen dem Akku (6) und der Biobrennstoffzelle (3) eingesetzten Energiewandler (7),
wobei der Energiewandler (7) so konfiguriert ist, dass er eine von der Biobrennstoffzelle (3) erzeugte elektrische Eingangsenergie empfängt und eine vorbestimmte elektrische Energie zum Laden des Akkus (6) abgibt, wobei optional die von dem Energiewandler abgegebene elektrische Energie größer ist als die elektrische Eingangsenergie,
wobei der Energiewandler (7) einen Spannungsregler enthält, der so konfiguriert ist, dass er als Eingang eine von der Biobrennstoffzelle (3) erzeugte elektrische Spannung empfängt und als Ausgang eine vorbestimmte elektrische Spannung, optional zwischen 2 V und 5,5 V, zum Laden des Akkus (6) abgibt, wobei optional die elektrische Ausgangsspannung von dem Spannungsregler höher ist als die elektrische Eingangsspannung.

10. Medizinprodukt nach einem der vorhergehenden Ansprüche, umfassend eine erste und eine zweite Nadel (11, 12), die geeignet sind, den Durchgang von Fluid des Patienten zu ermöglichen,
wobei die erste Nadel (11) in Fluidverbindung, optional direkt, mit der Pumpe (4) steht und so konfiguriert ist, dass sie Fluid aus dem Patienten entnimmt und dieses der Pumpe (4) zuführt,
wobei die zweite Nadel (12) in Fluidverbindung, optional direkt, mit der Biobrennstoffzelle (3) steht und so konfiguriert ist, dass sie eingehendes Fluid von der Biobrennstoffzelle (3) empfängt und zurück in den Patienten leitet.

11. Medizinprodukt nach einem der vorhergehenden Ansprüche, wobei die erste und zweite Nadel (11, 12) Mikronadeln sind, die so konfiguriert sind, dass sie den Durchgang von Blut und/oder interstitieller Flüssigkeit eines Patienten ermöglichen.

12. Medizinprodukt nach dem vorhergehenden Anspruch, wobei die mindestens eine erste Nadel (11) eine Anzahl von Mikronadeln aufweist, die größer als 50 ist, optional zwischen 100 und 5000, optional jede Mikronadel der mindestens einen ersten Nadel eine Länge zwischen 100 und 500 µm und/oder einen Durchmesser zwischen 5 und 50 µm aufweist,
wobei die mindestens eine zweite Nadel (12) eine Anzahl von mehr als 50 Mikronadeln aufweist, optional zwischen 100 und 5000, optional jede Mikronadel der mindestens einen zweiten Nadel eine Länge zwischen 100 und 500 µm und/oder einen Durchmesser zwischen 5 und 50 µm aufweist.

13. Medizinprodukt nach Anspruch 11 oder 12, wobei die Halterung (2) mindestens ein Pflaster umfasst, das mindestens eine Klebeschicht (2b) aufweist, die eine Kontaktfläche der Halterung (2) definiert, die für den direkten Kontakt mit der Haut eines Patienten geeignet ist, wobei sich die Halterung (2) in der Dicke zwischen der Kontaktfläche und einer gegenüberliegenden Halterungsoberfläche erstreckt, wobei die Halterung (2), optional das Pflaster, die Mikronadeln der ersten und zweiten Nadeln direkt trägt, wobei die Mikronadeln der mindestens einen ersten Nadel (11) über die Kontaktfläche der Halterung verteilt sind, optional gleichmäßig über die Kontaktfläche verteilt, mit einer Dichte größer als 50 Mikronadeln pro cm², optional mit einer Dichte zwischen 50 und 1000 Mikronadeln pro cm²,
wobei die Mikronadeln der mindestens einen zweiten Nadel (12) über die Kontaktfläche der Halterung verteilt sind, optional gleichmäßig über die Kontaktfläche verteilt, mit einer Dichte größer als 50 Mikronadeln pro cm², optional mit einer Dichte zwischen 50 und 1000 Mikronadeln pro cm².

14. Medizinprodukt nach einem der vorhergehenden Ansprüche, umfassend einen Ladeempfänger (16), der mit dem Akku (6) verbunden und zum Empfangen elektromagnetischer Wellen, optional Mikrowellen, von einem externen Ladegerät und zum Erzeugen elektrischer Energie aufgrund der elektromagnetischen Wellen konfiguriert ist, wobei der Ladeempfänger (16) dazu konfiguriert ist, elektrische Energie an den Akku (6) zu übertragen, um ihn aufzuladen.

15. Medizinisches System (100) umfassend:
- mindestens ein implantierbares Produkt (101), das unter der Haut eines Patienten (P) anwendbar ist, wobei das implantierbare Produkt einen Akku (102) enthält, der so konfiguriert ist, dass er das implantierbare Produkt selbst mit Strom versorgt,
- mindestens eine Ladequelle (104), die zum Bereitstellen elektrischer Energie an den Akku (102) des implantierbaren Produkts konfiguriert ist, wobei die Ladequelle mindestens ein Medizinprodukt (1) nach einem der vorhergehenden Ansprüche umfasst.

## Revendications

1. Dispositif médical (1) comprenant :
- un support (2) applicable sur la peau d'un patient (P),
- au moins une batterie (6) rechargeable portée par le support (2),
- au moins une pile à biocombustible (3) portée par le support (2) et connectée à la batterie (6), dans lequel la pile à biocombustible (3) est configurée pour recevoir un fluide du patient (P) et produire de l'énergie électrique pour recharger la batterie (6), **caractérisé par**
- au moins une pompe (4) portée par le support (2) et configurée pour acheminer le fluide du patient vers la pile à biocombustible (3),
- au moins un capteur (5) porté par le support (2) et configuré pour émettre un signal représentatif d'un paramètre de commande du fluide du patient,
- une unité de commande (50) connectée à la pompe (4) et au capteur (5) et active en commande sur ladite pompe (4),
dans lequel l'unité de commande (50) est configurée pour réaliser une procédure de génération de puissance comprenant les étapes suivantes :
∘ la réception d'un signal du capteur (5),
∘ le traitement du signal émis par le capteur (5) et l'estimation d'une valeur du paramètre de commande du fluide du patient,
∘ la comparaison de la valeur estimée du paramètre de commande à une valeur seuil, et
∘ sur la base de ladite comparaison, le fait d'ordonner l'activation de la pompe (4) pour acheminer le fluide du patient vers la pile à biocombustible (3) pour produire de l'électricité.

2. Dispositif médical selon la revendication 1, dans lequel ledit paramètre de commande inclut au moins l'un parmi : glycémie, hémoglobine glycatée, concentration en oxygène, fréquence cardiaque, tension artérielle, température, concentration en acides aminés ou en graisses.

3. Dispositif médical selon l'une quelconque des revendications précédentes, dans lequel le capteur (5) est un capteur optique de glucose, facultativement le capteur (5) inclut au moins un sélectionné dans le groupe de : un capteur infrarouge, un capteur de luminescence, un capteur de rayonnement électromagnétique, un capteur acoustique, un capteur à ultrasons, un circuit de mesure d'impédance, un capteur radiofréquence.

4. Dispositif médical selon l'une quelconque des revendications précédentes, dans lequel l'unité de commande (50), pendant l'étape d'estimation de la valeur du paramètre de commande de la procédure de génération de puissance, est configurée pour estimer la valeur de glucose dans le fluide du patient,
dans lequel l'unité de commande (50), pendant l'étape de comparaison ultérieure de la procédure de génération de puissance, est configurée pour comparer ladite valeur de glucose fluide à une valeur seuil, ladite valeur seuil étant facultativement comprise entre 60 mg/dl et 100 mg/dl.

5. Dispositif médical selon la revendication précédente, dans lequel l'étape d'activation de la pompe (4) de la procédure de génération de puissance est réalisée dans le cas où l'unité de commande (50) détermine une valeur de glucose estimée supérieure à la valeur seuil.

6. Dispositif médical selon l'une quelconque des revendications précédentes, dans lequel l'unité de commande (50) est connectée à la pile à biocombustible (3) qui est configurée pour émettre un signal représentatif d'un paramètre électrique représentatif de l'énergie électrique produite,
dans lequel la procédure de génération de puissance, réalisée par l'unité de commande (50), inclut également les étapes suivantes :
- la réception du signal de la pile à biocombustible (3),
- sur la base du signal émis par la pile à biocombustible (3), l'estimation d'une valeur du paramètre électrique,
- la comparaison de la valeur du paramètre électrique à une valeur seuil électrique,
- sur la base de ladite comparaison, le fait d'ordonner la transmission de l'énergie électrique produite par la pile à biocombustible (3) à la batterie (6),
dans lequel l'unité de commande (50) est configurée pour ordonner la transmission d'énergie électrique produite par la pile à biocombustible (3) à la batterie (6) lorsque la valeur du paramètre électrique est supérieure à la valeur seuil électrique.

7. Dispositif médical selon la revendication précédente, dans lequel le paramètre électrique inclut au moins l'un parmi : une tension électrique mesurée aux extrémités de la pile à biocombustible, un courant électrique, une énergie électrique, une résistance électrique.

8. Dispositif médical selon l'une quelconque des revendications précédentes, dans lequel la batterie (6) est connectée électriquement à la pompe (4), au capteur (5) et à l'unité de commande (50), dans lequel la batterie (6) est configurée pour fournir de l'énergie électrique pour alimenter la pompe (4), le capteur (5) et l'unité de commande (50).

9. Dispositif médical selon l'une quelconque des revendications précédentes comprenant un convertisseur d'énergie (7) interposé entre la batterie (6) et la pile à biocombustible (3), dans lequel ledit convertisseur d'énergie (7) est configuré pour recevoir en entrée l'énergie électrique produite par la pile à biocombustible (3) et émettre en sortie une énergie électrique prédéterminée pour charger la batterie (6), facultativement l'énergie électrique de sortie du convertisseur d'énergie est supérieure à l'énergie électrique d'entrée,
dans lequel le convertisseur d'énergie (7) inclut un régulateur de tension configuré pour recevoir en entrée une tension électrique générée par la pile à biocombustible (3) et émettre en sortie une tension électrique prédéterminée, facultativement comprise entre 2 V et 5,5 V, pour charger la batterie (6), facultativement la tension électrique de sortie du régulateur de tension est supérieure à la tension électrique d'entrée.

10. Dispositif médical selon l'une quelconque des revendications précédentes comprenant une première et une seconde aiguille (11, 12) adaptées pour permettre le passage de fluide du patient,
dans lequel la première aiguille (11) est en communication fluidique, facultativement directement, avec la pompe (4) et est configurée pour aspirer du fluide du patient et permettre de l'envoyer à la pompe (4),
dans lequel la seconde aiguille (12) est en communication fluidique, facultativement directement, avec la pile à biocombustible (3) et est configurée pour recevoir du fluide entrant de la pile à biocombustible (3) et le réintroduire dans le patient.

11. Dispositif médical selon l'une quelconque des revendications précédentes, dans lequel les première et seconde aiguilles (11, 12) sont des micro-aiguilles configurées pour permettre le passage de sang et/ou de fluide interstitiel d'un patient.

12. Dispositif médical selon la revendication précédente, dans lequel ladite au moins une première aiguille (11) présente un nombre de micro-aiguilles supérieur à 50, facultativement compris entre 100 et 5000, facultativement chaque micro-aiguille de ladite au moins une première aiguille présente une longueur comprise entre 100 et 500 µm et/ou un diamètre compris entre 5 et 50 mµ.
dans lequel ladite au moins une seconde aiguille (12) présente un nombre de micro-aiguilles supérieur à 50, facultativement compris entre 100 et 5000, facultativement chaque micro-aiguille de ladite au moins une seconde aiguille présente une longueur comprise entre 100 et 500 µm et/ou un diamètre compris entre 5 et 50 µm.

13. Dispositif médical selon la revendication 11 ou 12, dans lequel le support (2) comprend au moins un pansement ayant au moins une couche adhésive (2b) définissant une surface de contact du support (2) adaptée pour être directement en contact avec la peau d'un patient, dans lequel le support (2) s'étend en épaisseur entre la surface de contact et une surface de support opposée, dans lequel le support (2), facultativement le plâtre, porte directement les micro-aiguilles desdites première et seconde aiguilles, dans lequel les micro-aiguilles de ladite au moins une première aiguille (11) sont réparties sur la surface de contact du support, facultativement réparties uniformément sur ladite surface de contact, avec une densité supérieure à 50 micro-aiguilles par cm², facultativement avec une densité comprise entre 50 et 1000 micro-aiguilles par cm²,
dans lequel les micro-aiguilles de ladite au moins une seconde aiguille (12) sont réparties sur la surface de contact du support, facultativement réparties uniformément sur ladite surface de contact, avec une densité supérieure à 50 micro-aiguilles par cm², facultativement avec une densité comprise entre 50 et 1000 micro-aiguilles par cm².

14. Dispositif médical selon l'une quelconque des revendications précédentes comprenant un récepteur de charge (16) connecté à la batterie (6) et configuré pour recevoir des ondes électromagnétiques, facultativement des micro-ondes, d'un chargeur externe et générer de l'énergie électrique, en raison desdites ondes électromagnétiques, ledit récepteur de charge (16) étant configuré pour transmettre de l'énergie électrique à la batterie (6) pour la recharger.

15. Système médical (100) comprenant :
- au moins un dispositif implantable (101) applicable sous la peau d'un patient (P), dans lequel ledit dispositif implantable inclut une batterie (102) configurée pour alimenter le dispositif implantable lui-même,
- au moins une source de charge (104) configurée pour fournir de l'énergie électrique à la batterie (102) du dispositif implantable, dans lequel ladite source de charge comprend au moins un dispositif médical (1) selon l'une quelconque des revendications précédentes.
